# EUROPEAN PATENT SPECIFICATION

(11) **EP 1 002 116 B1**
(45) Date of publication and mention of the grant of the patent: **27.10.2004**
(21) Application number: 98937225.5
(22) Date of filing: 29.07.1998
(51) Int. Cl.: C12N 15/82, C12N 15/54, C12N 9/10, C12N 5/10, A01H 5/00

(54) **METHOD OF PRODUCING A DROUGHT-TOLERANT PLANT**
METHODE ZUR HERSTELLUNG EINER DÜRRE-RESISTENTEN PFLANZE
PROCEDE POUR LA PRODUCTION D' UNE PLANTE RESISTANTE A LA SECHERESSE

(30) Priority: 01.08.1997 US 4474 P
(43) Date of publication of application: 24.05.2000
(73) Proprietor: Performance Plants, Inc., Kingston, Ontario K7L 3N6 (CA)
(72) Inventor: McCOURT, Peter, Toronto, Ontario M6G 4A3 (CA); GHASSEMIAN, Majid, San Diego, California 92129 (US); CUTLER, Sean, Lewiston, New York 14092-1909 (US); BONETTA, Dario, Toronto, Ontario M4Y 1G1 (CA)
(74) Representative: White, Martin Paul
(86) International application number: PCT/US1998/015664
(87) International publication number: WO 1999/006580

(56) References cited:
- CUTLER, S.R., ET AL. : "UNTITLED" EMBL SEQUENCE DATA LIBRARY, 14 February 1996, XP002090869 HEIDELBERG, GERMANY
- CUTLER, S.R., ET AL. : "untitled" EMBL SEQUENCE DATA LIBRARY, 1 November 1996, XP002090870 heidelberg, germany
- YANG Z ET AL: "PROTEIN FARNESYLTRANSFERASE IN PLANTS 1 MOLECULAR CLONING AND EXPRESSION OF A HOMOLOG OF THE BETA SUBUNIT FROM THE GARDEN PEA" PLANT PHYSIOLOGY, vol. 101, no. 2, February 1993, pages 667-674, XP000647522
- CUTLER, S., ET AL. : "a protein farnesyl transferase involved in abscisic acid signal transduction in arabidopsis" SCIENCE, vol. 273, 30 August 1996, pages 1239-1241, XP002090871 cited in the application
- MERLOT, S. AND GIRAUDAT, J.: "genetic analysis of abscisic acid signal transduction" PLANT PHYSIOLOGY, vol. 114, July 1997, pages 751-757, XP002090872
- QIAN, D., ETAL. : "protein farnesyltransferase in plants: molecular characterization and involvement in cell cycle control" THE PLANT CELL, vol. 8, December 1996, pages 2381-2394, XP002090873
- PEI,Z-M., ET AL. : "role of farnesyltransferase in ABA regulation of guard cell anion channels and plant water loss" SCIENCE, vol. 282, 9 October 1998, pages 287-290, XP002090874

## Description

### BACKGROUND OF THE INVENTION

Most higher plants encounter at least transient decreases in relative water content at some stage of their life cycle and, as a result, have evolved a number of desiccation protection mechanisms. If however, the change in water deficit is prolonged the effects on the plant's growth and development can be profound. Decreased water content due to drought, cold or salt stresses can irreparably damage plant cells which in turn limits plant growth and crop productivity in agriculture.

Plants respond to adverse conditions of drought, salinity and cold with a variety of morphological and physiological changes. Although our understanding of plant tolerance mechanisms to these stresses is fragmentary, the plant hormone abscisic acid (ABA) has been proposed to be an essential mediator between environmental stimulus and plant responses. ABA levels increase in response to water deficits and exogenously applied ABA mimics many of the responses normally induced by water stress. Once ABA is synthesized it causes the closure of the leaf stomata thereby decreasing water loss through transpiration.

The identification of genes that transduce ABA into a cellular response opens the possibility of exploiting these regulators to enhance desiccation tolerance in crop species. In principle, these ABA signalling genes can be coupled with the appropriate controlling elements to allow optimal plant growth and development. Thus, not only would these genes allow the genetic tailoring of crops to withstand transitory environmental insults, they should also broaden the environments where traditional crops can be grown.

In addition, little is known of the genetic mechanisms which control plant growth and development. Genes which further affect other metabolic processes such as senescence and growth habits of plants can be useful in a wide variety of crop and horticultural plants.

### SUMMARY OF THE INVENTION

This invention relates to a method for enhancing the drought tolerance of plants as defined in the appended claims. Further, this invention finds utility in the control of regulatory functions in photosynthetic organisms; for example, in the control of growth habit, flowering, seed production, seed germination, and senescence in such organisms.

This invention is based on a method for enhancing the drought tolerance of plants by means of alterations in isolated or recombinant nucleic acids encoding a farnesyl transferase (Ftase) protein or its functional equivalent.

This invention also relates to a transgenic plant obtainable by a method of the invention and a transgenic seed obtainable by the transgenic plant.

### BRIEF DESCRIPTION OF THE DRAWINGS

Figures 1A-1C show the nucleic acid sequence of the ERA1 gene (SEQ ID NO:1) in which the introns are underlined and the start codon (ATG) is at nucleotide positions 1-3.
Figure 2 is the amino acid sequence of the ERA1 protein (SEQ ID NO:2).
Figures 3A-3B show the nucleic acid sequence of the ERA1 promoter (SEQ ID NO:3) .
Figure 4 is the amino acid sequence of the β subunit farnesylation domain from *Arabidopsis* (Arab.) (SEQ ID NO:2) aligned with the β subunit farnesylation domains from pea (SEQ ID NO:4), yeast (SEQ ID NO:5) and rat (SEQ ID NO:6) Residues that are identical to the *Arabidopsis* sequence are indicated with a dot. A dash indicates a blank. The amino acid positions of the *Arabidopsis* gene are indicated on the right-hand side.
Figure 5 is a photograph of an *era*1-transformed *Arabidopsis* plant (right) compared to the wild-type (control; *i*.*e*., naturally-occurring) plant (left) under extremely dry conditions.
Figure 6 is a graph comparing the water consent of *Arabidopsis* plants with inactivated or mutant Ftase activity (M. Columbia, *era* 1-2) and controls (M.C. control, *era* 1-2 control).
Figure 7 is a graph comparing the rate of water loss for the *Arabidopsis* plants with inactivated or mutant Ftase activity (*M*. *Columbia*, *era* 1-2) and controls (M.C. control, *era* 1-2 control).
Figure 8 is a comparison of aging leaves from control (wild-type) and *era*-2 mutant plants.
Figure 9 is a comparison of transcript levels in aging leaves from control (wild-type) and *era*-2 mutant plants.

### DETAILED DESCRIPTION OF THE INVENTION

This invention relates to a method as defined in the attached claims.

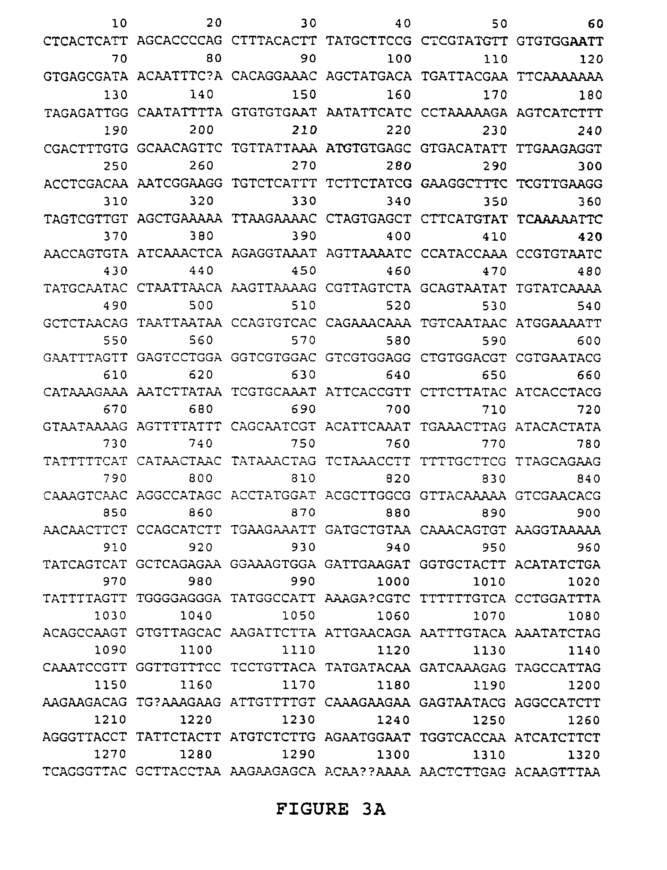

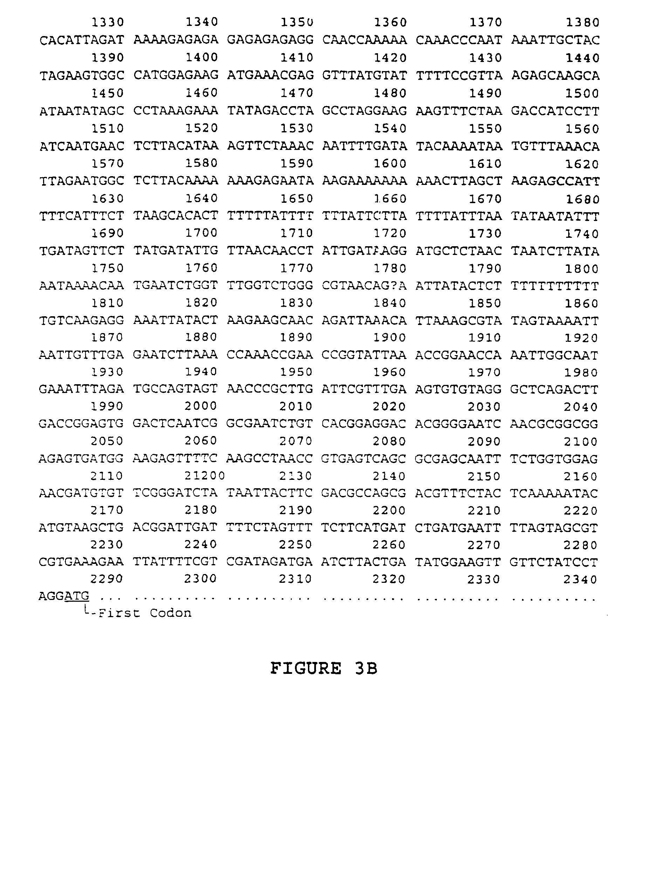

When incorporated into a plant, the ERA1 promoter is regulated in the guard cells of the plant and can affect water loss through the stomates. This promoter consists of a nucleic acid comprising SEQ ID NO:3 (Figure 3).

Transgenic plants and transgenic seeds obtainable this method are also part of this invention. The method can be used for producing a gene product under the control of a promoter which operates primarily in guard cells through expression of a gene encoding the gene product in the cell of a plant comprising the steps of: transforming a plant cell with a DNA construct comprising a) a regulatory region comprising SEQ ID NO:3 or a functional portion thereof, DNA comprising a structural gene encoding a gene product, and a 3' untranslated region containing a polyadenylated region; regenerating a plant, photosynthetic organism or tissue culture from the cell; and placing the plant, photosynthetic organisms or tissue culture under conditions so that the promoter induces transcription of the structural gene and the gene product is expressed.

In the context of this disclosure, the terms "regulatory region" or "promoter" refer to a sequence of DNA, usually upstream (5') to the coding sequence of a structural gene, which controls the expression of the coding region by providing recognition and binding sites for RNA polymerase and/or other factors required for transcription to start at the correct site. The term "functional portion" or "functional fragment" refers to a truncated sequence of a promoter of this invention which maintains the capability of inducing transcription of an ERA structural gene under the conditions described for activity of an Ftase protein.

The constructs and methods described herein can be applied to all types of plants and other photosynthetic organisms, including, but not limited to: angiosperms (monocots and dicots), gymnosperms, spore-bearing or vegetatively-reproducing plants and the algae, including the cyanophyta (blue-green algae). Particularly preferred plants are those plants which provide commercially-valuable crops, such as corn, wheat, cotton, rice, canola, sugar cane, sugar beet, sunflowers, potatoes, tomatoes, broccoli, carrots, lettuce, apple, plum, orange, lemon, rose, and the like.

Further, the constructs and methods described herein can be adapted to any plant part, protoplast, or tissue culture wherein the tissue is derived from a photosynthetic organism. The term "plant part" is meant to include a portion of a plant capable of producing a regenerated plant. Preferable plant parts include roots and shoots and meristematic portions thereof. Other plant parts encompassed by this invention are: leaves, flowers, seeds, epicotyls, hypocotyls, cotyledons, cotyledonary nodes, explants, pollen, ovules, meristematic or embryonic tissue, protoplasts, and the like. Transgenic plants can be regenerated from any of these plant parts, including tissue culture or protoplasts, and also from explants. Methods will vary according to the species of plant.

Compositions and constructs comprising isolated nucleic acids (both DNA and RNA) encoding an Ftase and portions thereof of photosynthetic organisms are described herein. Compositions and constructs comprising isolated nucleic acids encoding an Ftase promoter are also described herein. In particular, the ERA1 gene encoding the β subunit of Ftase from *Arabidopsis* and a regulatory sequence which regulates the transcription of the ERA1 gene have been isolated and sequenced. Nucleic acids which encode Ftases from photosynthetic organisms, and homologues or analogs of these nucleic acids, are described.

Methods using isolated and/or recombinant nucleic acids (DNA or RNA) are described. The nucleic acids are characterized by their ability to hybridize to (a) a nucleic acid encoding an Ftase protein or polypeptide, such as a nucleic acid having the sequences of SEQ ID NO:1 or (b) a portion of the foregoing (*e.g.*, a portion comprising the minimum nucleotides required to encode a functional Ftase protein; or by the ability to encode a polypeptide having the amino acid sequence of an Ftase (*e*.*g*., SEQ ID NO:2, or to encode functional equivalents thereof; *e*.*g*., a polypeptide having at least 80% sequence similarity to SEQ ID NO:2, which when incorporated into a plant cell, facilitates the growth habit, seed germination, and metabolism in a photosynthetic organism in the same manner as SEQ ID NO:1). A functional equivalent of an Ftase therefore, would have at least an 80% similar amino acid sequence and similar characteristics to, or perform in substantially the same way as, the polypeptide encoded by SEQ ID NO:2. A nucleic acid which hybridizes to a nucleic acid encoding an Ftase polypeptide such as SEQ ID NO:2 can be double- or single-stranded. Hybridization to DNA such as DNA having the sequence SEQ ID NO:1, includes hybridization to the strand shown or its complementary strand.

The percent amino acid sequence similarity between an Ftase polypeptide such as SEQ ID NO:2, and functional equivalents thereof is at least about 60% (≥ 60%); or the percent amino acid sequence similarity between an Ftase polypeptide and its functional equivalents is at least about 75% (≥75%); or the percent amino acid sequence similarity between an Ftase polypeptide and its functional equivalents is at least about 80%, or at least about 90%, when consecutive amino acids are compared.

Isolated and/or recombinant nucleic acids meeting these criteria comprise nucleic acids having sequences identical to sequences of naturally occurring ERA1 genes and portions thereof, or variants of the naturally occurring genes. Such variants include mutants differing by the addition, deletion or substitution of one or more nucleotides, modified nucleic acids in which one or more nucleotides are modified (e.g., DNA or RNA analogs), and mutants comprising one or more modified nucleotides.

Such nucleic acids, including DNA or RNA, can be detected and isolated by hybridization under high stringency conditions or moderate stringency conditions, for example, which are chosen so as to not permit the hybridization of nucleic acids having non-complementary sequences. "Stringency conditions" for hybridizations is a term of art which refers to the conditions of temperature and buffer concentration which permit hybridization of a particular nucleic acid to another nucleic acid in which the first nucleic acid may be perfectly complementary to the second, or the first and second may share some degree of complementarity which is less than perfect. For example, certain high stringency conditions can be used which distinguish perfectly complementary nucleic acids from those of less complementarity. "High stringency conditions" and "moderate stringency conditions" for nucleic acid hybridizations are explained on pages 2.10.1-2.10.16 (see particularly 2.10.8-11) and pages 6.3.1-6 in *Current Protocols in Molecular Biology* (Ausubel, F.M. *et al*., eds., Vol. 1, containing supplements up through Supplement 29, 1995). The exact conditions which determine the stringency of hybridization depend not only on ionic strength, temperature and the concentration of destabilizing agents such as formamide, but also on factors such as the length of the nucleic acid sequence, base composition, percent mismatch between hybridizing sequences and the frequency of occurrence of subsets of that sequence within other non-identical sequences. Thus, high or moderate stringency conditions can be determined empirically.

High stringency hybridization procedures can (1) employ low ionic strength and high temperature for washing, such as 0.015 M NaCl/ 0.0015 M sodium citrate, pH 7.0 (0.1x SSC) with 0.1% sodium dodecyl sulfate (SDS) at 50°C; (2) employ during hybridization 50% (vol/vol) formamide with 5x Denhardt's solution (0.1% weight/volume highly purified bovine serum albumin/ 0.1% wt/vol Ficoll/ 0.1% wt/vol polyvinylpyrrolidone), 50 mM sodium phosphate buffer at pH 6.5 and 5x SSC at 42°C; or (3) employ hybridization with 50% formamide, 5x SSC, 50 mM sodium phosphate (pH 6.8), 0.1% sodium pyrophosphate, 5x Denhardt's solution, sonicated salmon sperm DNA (50 µg/ml), 0.1% SDS, and 10% dextran sulfate at 42°C, with washes at 42°C in 0.2x SSC and 0.1% SDS. Moderate stringency conditions would be similar except that hybridization would employ 25% formamide in place of 50% formamide.

By varying hybridization conditions from a level of stringency at which no hybridization occurs to a level at which hybridization is first observed, conditions which will allow a given sequence to hybridize with the most similar sequences in the sample can be determined.

Exemplary conditions are described in Krause, M.H. and S.A. Aaronson (1991) *Methods in Enzymology, 200*:546-556. Also, see especially page 2.10.11 in *Current Protocols in Molecular Biology* (*supra*), which describes how to determine washing conditions for moderate or low stringency conditions. Washing is the step in which conditions are usually set so as to determine a minimum level of complementarity of the hybrids. Generally, from the lowest temperature at which only homologous hybridization occurs, a 1% mismatch between hybridizing nucleic acids results in a 1°C decrease in the melting temperature Tₘ, for any chosen SSC concentration. Generally, doubling the concentration of SSC results in an increase in Tₘ of -17°C. Using these guidelines, the washing temperature can be determined empirically for moderate or low stringency, depending on the level of mismatch sought.

Isolated and/or recombinant nucleic acids that are characterized by their ability to hybridize to (a) a nucleic acid encoding an Ftase polypeptide, such as the nucleic acids depicted as SEQ ID NO:1, (b) the complement of SEQ ID NO:1, (c) or a portion of (a) or (b) (*e*.*g*. under high or moderate stringency conditions), may further encode a protein or polypeptide having at least one functional characteristic of an Ftase polypeptide, such as regulation of lateral branching under diurnal light cycles, or regulation of the response to ABA, or regulation of senescence.

Enzymatic assays, complementation tests, or other suitable methods can also be used in procedures for the identification and/or isolation of nucleic acids which encode a polypeptide such as a polypeptide of the amino acid sequence SEQ ID NO:2 or a functional equivalent of this polypeptide. The antigenic properties of proteins or polypeptides encoded by hybridizing nucleic acids can be determined by immunological methods employing antibodies that bind to an Ftase polypeptide such as immunoblot, immunoprecipitation and radioimmunoassay. PCR methodology, including RAGE (Rapid Amplification of Genomic DNA Ends), can also be used to screen for and detect the presence of nucleic acids which encode Ftase-like proteins and polypeptides, and to assist in cloning such nucleic acids from genomic DNA. PCR methods for these purposes can be found in Innis, M.A., *et al*. (1990) *PCR Protocols: A Guide to Methods and Applications,* Academic Press, Inc., San Diego, CA.

The nucleic acids described herein are used in the methods of the present invention for production of proteins or polypeptides which are incorporated into cells, tissues, plant parts, plants and other photosynthetic organisms. In one embodiment, DNA containing all or part of the coding sequence for an Ftase polypeptide, or DNA which hybridizes to DNA having the sequence SEQ ID NO:2 is incorporated into a vector for expression of the encoded polypeptide in suitable host cells. The encoded polypeptide consisting of an Ftase subunit or its functional equivalent is capable of farnesyl transferase activity. The term "vector" as used herein refers to a nucleic acid molecule capable of transporting another nucleic acid to which it has been linked.

Primers and probes consisting of 20 or more contiguous nucleotides of the above-described nucleic acids are also described herein. Thus, one nucleic acid comprises a specific sequence of about 20 to about 200 or more nucleotides which are identical or complementary to a specific sequence of nucleotides of the Ftase protein-encoding DNA or transcribed mRNA. These probes and primers can be used to identify and isolate Ftase-encoding nucleic acid from other photosynthetic organisms.

Nucleic acids referred to herein as "isolated" are nucleic acids separated away from the nucleic acids of the genomic DNA or cellular RNA of their source of origin (e.g., as it exists in cells or in a mixture of nucleic acids such as a library), and may have undergone further processing. "Isolated" nucleic acids include nucleic acids obtained by methods described herein, similar methods or other suitable methods, including essentially pure nucleic acids, nucleic acids produced by chemical synthesis, by combinations of biological and chemical methods, and recombinant nucleic acids which are isolated. Nucleic acids referred to herein as "recombinant" are nucleic acids which have been produced by recombinant DNA methodology, including those nucleic acids that are generated by procedures which rely upon a method of artificial recombination, such as the polymerase chain reaction (PCR) and/or cloning into a vector using restriction enzymes. "Recombinant" nucleic acids are also those that result from recombination events that occur through the natural mechanisms of cells, but are selected for after the introduction to the cells of nucleic acids designed to allow or make probable a desired recombination event. Portions of the isolated nucleic acids which code for polypeptides having a certain function can be identified and isolated by, for example, the method of Jasin, M., *et al*., U.S. Patent No. 4,952,501.

The invention uses antisense nucleic acids or oligonucleotides which are complementary, in whole or in part, to a target molecule comprising a sense strand, and can hybridize with the target molecule. The target can be DNA, or its RNA counterpart (*i*.*e*., wherein T residues of the DNA are U residues in the RNA counterpart). When introduced into a cell, antisense nucleic acids or oligonucleotides can inhibit the expression of the gene encoded by the sense strand or the mRNA transcribed from the sense strand. Antisense nucleic acids can be produced by standard techniques. See, for example, Shewmaker, *et al*., U.S. Patent No. 5,107,065.

In a particular embodiment, an antisense nucleic acid or oligonucleotide is wholly or partially complementary to and can hybridize with a target nucleic acid (either DNA or RNA), wherein the target nucleic acid can hybridize to a nucleic acid having the sequence of the complement of the strand in SEQ ID NO:1. For example, an antisense nucleic acid or oligonucleotide can be complementary to a target nucleic acid having the sequence shown as the strand of the open reading frame of SEQ ID NO:1, or nucleic acid encoding a functional equivalent of Ftase, or to a portion of these nucleic acids sufficient to allow hybridization. A portion, for example, a sequence of 16 nucleotides could be sufficient to inhibit expression of the protein. Fragments comprising 25 or more consecutive nucleotides complementary to SEQ ID NO:1 could also be used. Or, an antisense nucleic acid or oligonucleotide complementary to 5' or 3' untranslated regions, or overlapping the translation initiation codon (5' untranslated and translated regions), of the ERA1 gene, or a gene encoding a functional equivalent can also be effective. In another embodiment, the antisense nucleic acid is wholly or partially complementary to and can hybridize with a target nucleic acid which encodes an Ftase polypeptide.

In addition to the antisense nucleic acids, oligonucleotides can be constructed which will bind to duplex nucleic acid either in the gene or the DNA:RNA complex of transcription, to form a stable triple helix-containing or triplex nucleic acid to inhibit transcription and/or expression of a gene encoding an Ftase polypeptide or its functional equivalent. Frank-Kamenetskii, M.D. and Mirkin, S.M. (1995) *Ann*. *Rev*. *Biochem*. *64*:65-95. Such oligonucleotides are constructed using the base-pairing rules of triple helix formation and the nucleotide sequence of the gene or mRNA for Ftase. These oligonucleotides can block Ftase-type activity in a number of ways, including prevention of transcription of the ERA1 gene or by binding to mRNA as it is transcribed by the gene.

Proteins or polypeptides are encoded by the nucleic acids described herein. The proteins and polypeptides can be isolated and/or recombinant. Proteins or polypeptides referred to herein as "isolated" are proteins or polypeptides purified to a state beyond that in which they exist in cells. They are at least 10% pure; *i.e.*, substantially purified. "Isolated" proteins or polypeptides include proteins or polypeptides obtained by methods described *infra*, similar methods or other suitable methods, and include essentially pure proteins or polypeptides, proteins or polypeptides produced by chemical synthesis or by combinations of biological and chemical methods, and recombinant proteins or polypeptides which are isolated. Proteins or polypeptides referred to herein as "recombinant" are proteins or polypeptides produced by the expression of recombinant nucleic acids.

The protein or portion thereof has at least one function characteristic of an Ftase; for example, catalytic activity affecting, *e*.*g*., normal lateral branching, florets/inflorescence, seed germination, or stomatal opening, and binding function, and/or antigenic function (e.g., binding of antibodies that also bind to naturally occurring Ftase). As such, these proteins are referred to as Ftases of plant origin, and include, for example, naturally occurring Ftase, variants (e.g. mutants) of those proteins and/or portions thereof. Such variants include mutants differing by the addition, deletion or substitution of one or more amino acid residues, or modified polypeptides in which one or more residues are modified, and mutants comprising one or more modified residues.

The invention uses a nucleic acid sequence encoding the β subunit of an Ftase protein. Portions of the enzyme can be made which have full or partial function on their own, or which when mixed together (though fully, partially, or nonfunctional alone), spontaneously assemble with one or more other polypeptides to reconstitute a functional protein having at least one functional characteristic of an Ftase.

A number of genes have been identified that are induced by ABA. This suggests that ABA-induced tolerance to adverse environmental conditions is a complex multigenic event. Thus, identification and transfer of single genes into crop plants which improves the viability of the plant under different environmental conditions due to increased responsiveness to ABA is novel and extremely useful.

To identify genes that could be more global controllers of ABA-regulated plant processes, genetic screens were applied in a number of plant species to isolate mutations that alter the response of the plant to the hormone.

Mutations that confer enhanced response to ABA (era) in *Arabidopsis* seeds were identified by their ability to prevent seed germination with low concentrations of ABA that normally permit wild-type (controls, *i.e.*, naturally-occurring) seed germination. Of these, the eral mutant class, which includes one transferred DNA (T-DNA) line (*era*l-1, ecotype Wassilewskija) and two neutron-generated mutants (*era*1-2 and *era*1-3, ecotype Columbia), was of added interest because this class showed decreased germination efficiency under normal postimbibition. Mutations that enhance ABA responsiveness should, in principle, be more dormant. Dormancy in eral alleles was alleviated by a 4-day chilling period; the efficiency of *era*1 germination increased with the length of time the seeds are chilled. In many plant species, breaking dormancy to allow germination requires vernalization and exposure to moist, low-temperature environments for an extended period (Baskin and Baskin, 1971). The germination profile of *era* mutants could reflect an increased state of ABA-induced dormancy; consequently, these seeds require longer vernalization to germinate. Support for this contention came from construction of double mutants of *era1* with both ABA biosynthetic (*aba1-1*) and insensitive mutants (*abi1-1* and *abi3-6*). In all cases, the double mutants had reduced dormancy as compared with *era*1, indicating that the increased dormancy observed in *era*1 seed was dependent on ABA synthesis or sensitivity.

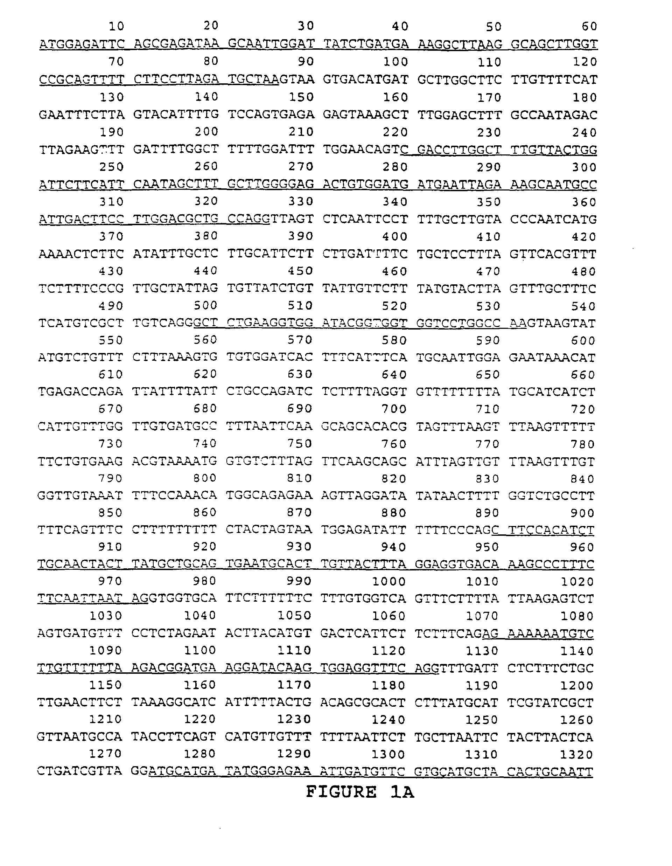

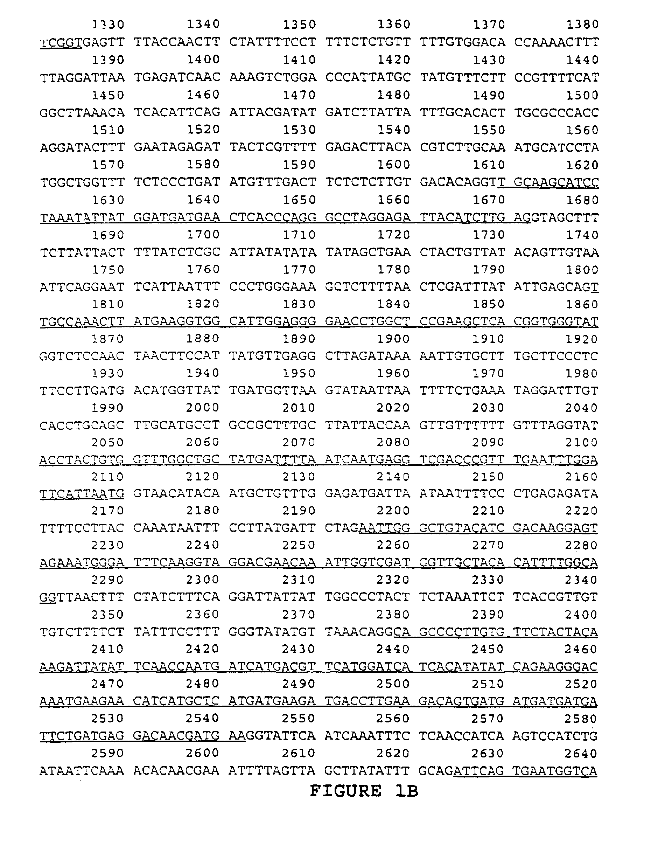

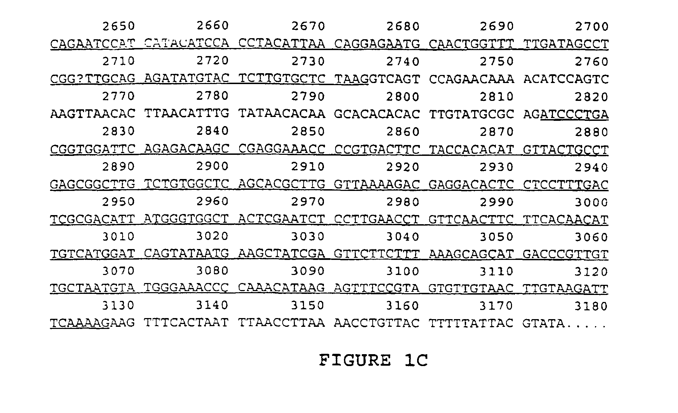

Aside from broadening the spectrum of new ABA response mutants, supersensitivity screens were also used to identify negative regulators of ABA sensitivity. That is, inhibition of these gene functions enhances the ABA response. One of these genes (ERA1) has been cloned and demonstrated to encode the β-subunit of a heterodimeric protein farnesyl transferase (Ftase) (Cutler *et al*., 1996). The *era*1-1 mutation, which is due to a T-DNA insertion, allowed the isolation of plant genomic regions flanking the insertions. Using the flanking regions as probes, the wild-type cDNA and genomic clones were isolated. Sequence analysis of these described a gene encompassing 3.5 kb of genomic DNA. The gene contains 13 introns which are underlined in Figures 1A-1C and the T-DNA insertion site in *era1-1* is in intron 8. Southern (DNA) analysis of wild-type DNA, *era1-2*, and *era1-3* probed with *Era1*cDNA revealed that both fast-neutron alleles contain deletions spanning the *ERA1* locus. Fast-neutron mutagenesis induced small deletions in *Arabidopsis* (Shirley *et al*., 1992), and subsequent genomic analysis with a 14-kb probe that spans the *ERA*1 locus determined the size of the *era*1-2 deletion to be about 7.5 kb and the *era*1-3 deletion to be slightly larger. Thus all three eral alleles contained DNA disruptions at the same locus, confirming the identity of the ERA locus.

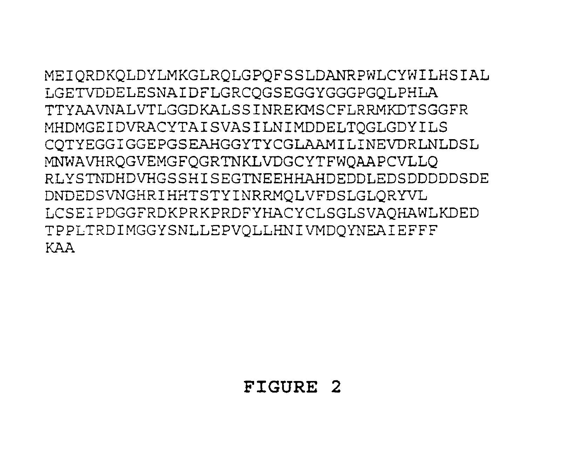

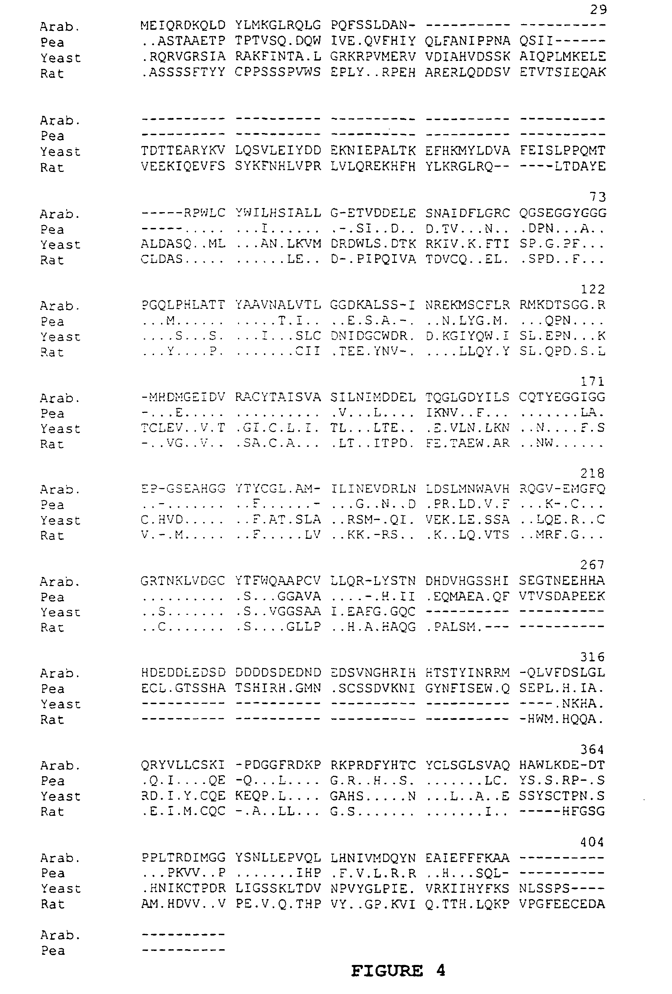

Conceptual translation of the longest open reading frame (404 amino acids) in the ERA1 gene produced a protein (Figures 2 and 4) with a high sequence similarity to yeast, pea, and mammalian protein farnesyl transferase β subunit genes (Goodman *et al*., 1988; Chen *et al*., 1991; Yang *et al*., 1993). Farnesyl transferases consist of α and β subunits that dimerize, forming an enzyme that catalyzes the attachment of farnesyl pyrophosphate (15 carbons) to proteins containing a COOH-terminal CaaX motif (Schafer and Rine, 1992), where C designates cysteine residue, aa is usually aliphatic amino acids, and X may designate a cysteine, serine, methionine, or glutamine residue. Both plant β subunit genes contain a region of about 50 amino acids near their COOH-terminus that is absent in yeast and animal β subunit genes.

In yeast and mammalian systems, Ftases modify several signal transduction proteins for membrane localization. This is achieved by the attachment of the lipophilic farnesyl sidechain to the protein target via the Ftase. The attachment of the farnesyl group causes a change in the overall hydrophobicity of the target allowing the protein to anchor itself into the membrane where it usually interacts with other signal transduction molecules. That the loss of farnesylation activity in the eral mutant leads to an enhanced response of the seed to ABA suggests a target protein in *Arabidopsis* must be localized to the membrane to attenuate the ABA signal. Thus farnesylation in *Arabidopsis*, appears to be required for the normal function of a negative regulator of ABA sensitivity.

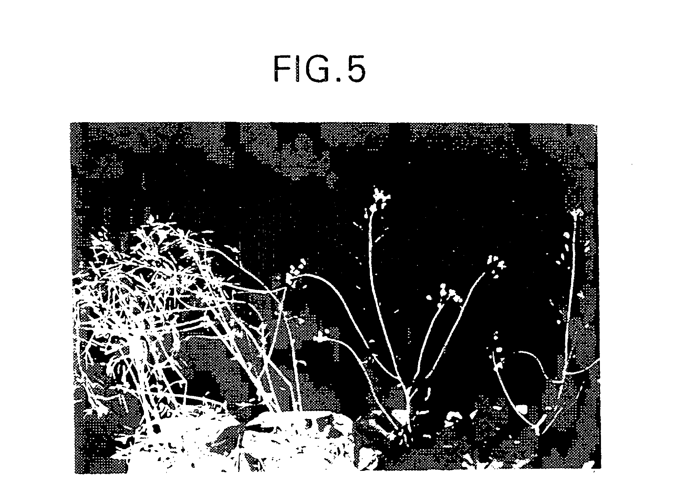

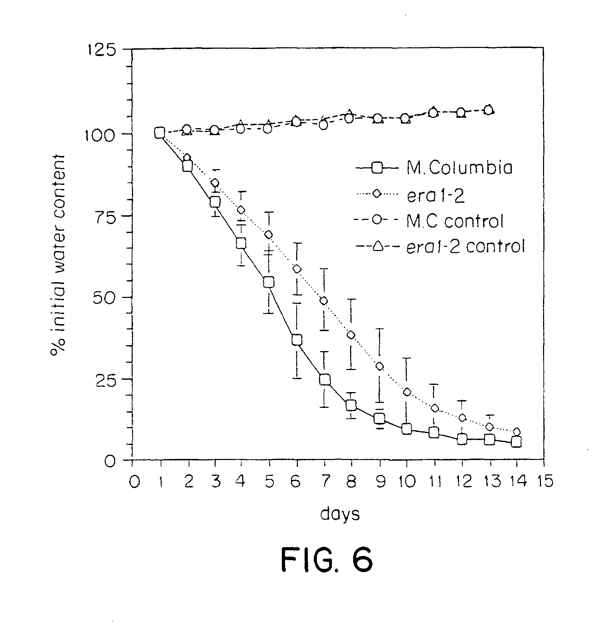

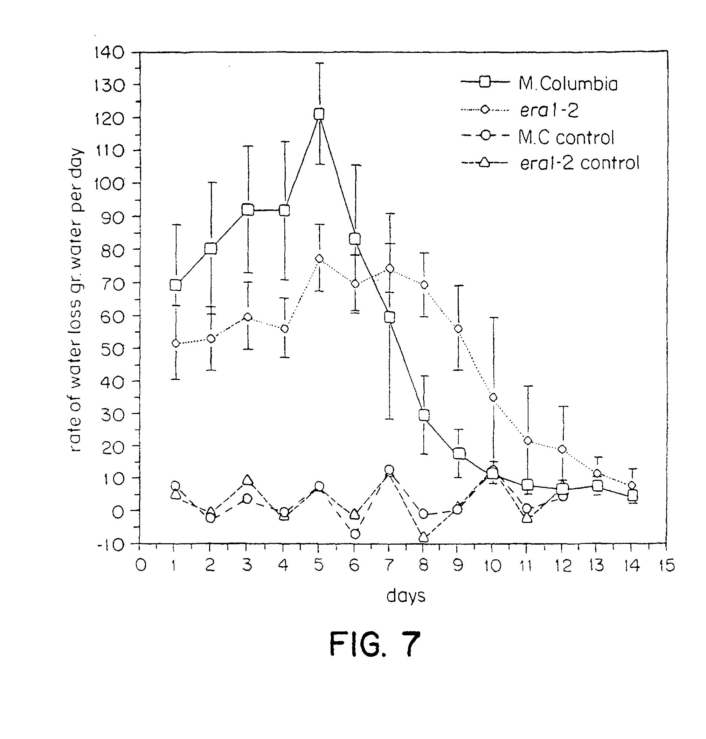

Subsequent work has shown that loss of ERA1 gene function in *Arabidopsis* confers an enhanced tolerance to environmental stresses at the level of the mature plant. For example, a comparison of wild-type plants and eral mutant plants grown in soil under standard laboratory conditions (24 hr light, 150 µE m⁻²sec⁻¹, 30% humidity) showed that the mutants did not require water as frequently as the wild-type plants in order to maintain viability (Figure 5). When mutant and wild-type plants were grown until flowering occurred, watering was stopped and the plants were observed each subsequent day for signs of stress. Water loss was significantly reduced in the mutant plants compared to the wild-type plants (Figures 6 and 7).

To determine if the observed increased drought tolerance of era mutants was related to ERA1 gene function, transgenic plants containing a ERA1 promoter fusion to a reporter GUS gene (made by inserting a 5 Kb fragment of the ERA1 promoter into a promoterless GUS T-DNA plasmid), were constructed. Analysis of the transgenic plants showed that ERA1 is transcriptionally expressed in the epidermal tissue of *Arabidopsis* and that this expression is guard-cell specific. Expression of ERA1 was also noted in the meristematic tissue of the plants and in root hairs. The guard cell expression of ERA1 is consistent with the drought tolerance of the mutant as these cells are the major regulators of water transpiration through the plant. It would be expected that ERAl-regulated stomatal conductance would require expression of the ERA1 gene in the guard cells. Hence loss of ERA1 gene function results in guard cells which are more responsive to ABA which, in turn, leads to more drought responsive guard cell regulation. Therefore, modification of Ftase expression or activity in higher plants, especially crop plants, will have profound effects on stomatal conductance and transpiration rates in the plants.

The nature of the *era*1 mutation in *Arabidopsis* demonstrates chat inhibition of farnesylation will enhance ABA responses in a plant and alteration of this enzyme activity in crop species. Inhibition of Ftase activity in crop plants can be achieved via a number of methods. For example, antisense technology of cognate ERA1 genes in a variety of crop species can be used to reduce Ftase activity, thus increasing drought tolerance. By specifically producing ERA1 antisense RNA in guard cells, the amount of Ftase synthesized can be reduced to a level which would mimic era mutant phenotypes. The ERA1 promoter is regulated in a number of different tissues ranging from shoot meristems to root hairs. By determining the elements of the ERA1 promoter which allow expression in specific tissues, it is possible to tailor the expression of antisense ERA1 to only one tissue or cell type, such as guard cells.

Another method to inhibit Ftase activity in plants is the production of specific peptide inhibitors of farnesylation in transgenic plants. In mammalian and yeast systems, the carboxyl terminal target sequence (CaaX, where C=cysteine, x=aliphatic, X=any amino acid) which allows the attachment of the farnesyl group to specific proteins has been clearly defined. Peptides which mimic these target sequences have been made and shown to inhibit farnesylation of the endogenous target proteins in these systems. Moreover, CAIM is farnesylated *in vivo* in *Arabidopsis*. Thus, similar inhibitors can be applied to higher plants to competitively inhibit Ftase *in vivo*. Again, this can be done through expression of inhibitor peptides in transgenic plants by synthesizing the DNA sequence for a CaaX peptide and fusing it to a guard cell-specific promoter. In both methods, using the appropriate promoters, antisense Ftase or peptide inhibitors can be specifically targeted and controlled.

Thus, this invention provides a method of producing drought-tolerant plants comprising: preparing a nucleic acid construct which comprises a promoter operably-linked to a nucleic acid comprising or encoding antisense to SEQ ID NO:1, or nucleic acid comprising a functional equivalent of the antisense; inserting the nucleic acid construct into a vector; transforming a plant, tissue culture, or plant cells with the vector; and growing the plant or regenerating a plant from the tissue culture or plant cells; wherein drought-tolerant plants are produced. This method can be used wherein the nucleic acid is selected from the group consisting of 25-200 or more consecutive nucleotides complementary to SEQ ID NO:1, oligonucleotides consisting of 25 or more consecutive nucleotides of SEQ ID NO:1 or its complement, or nucleic acid encoding a peptide inhibitor of farnesyl transferase

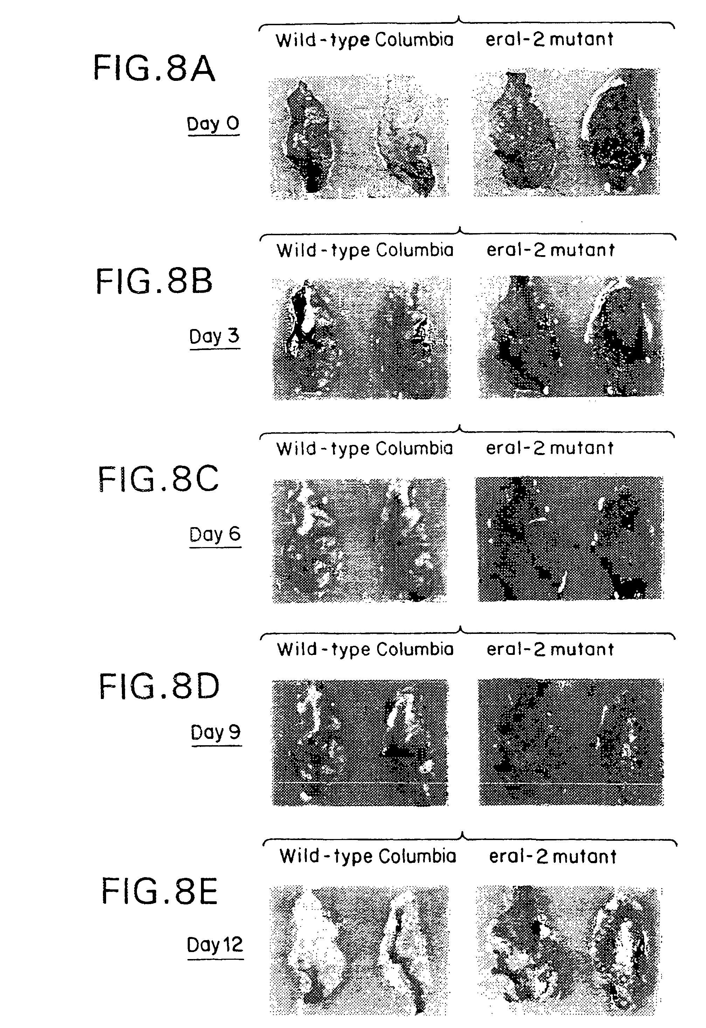

In addition to stomatal regulation which is extremely sensitive to ABA, era plants also demonstrate delayed senescence under drought conditions, indicating that farnesylation negatively regulates a number of drought-induced responses in *Arabidopsis*. The era plants grown under normal laboratory conditions take longer to turn yellow. The mutant plants remained green and viable long after the wild-type had senesced and died. Detached leaves of an era mutant plant do not yellow as quickly as detached leaves of wild-type plants (Figure 8). Similar-sized leaves which were developmentally identical were taken from wild-type and *era* plants and placed on agar-containing petri plates (See Example 7). Normally, a wild-type leaf begins to lose chlorophyll about five days later and eventually bleachs. The leaves of the mutant plants remained green for twice as long. Because the leaves were in constant contact with the agar they were not drought stressed, indicating the reduced senescence of the eral mutant is not a drought-induced phenomenon.

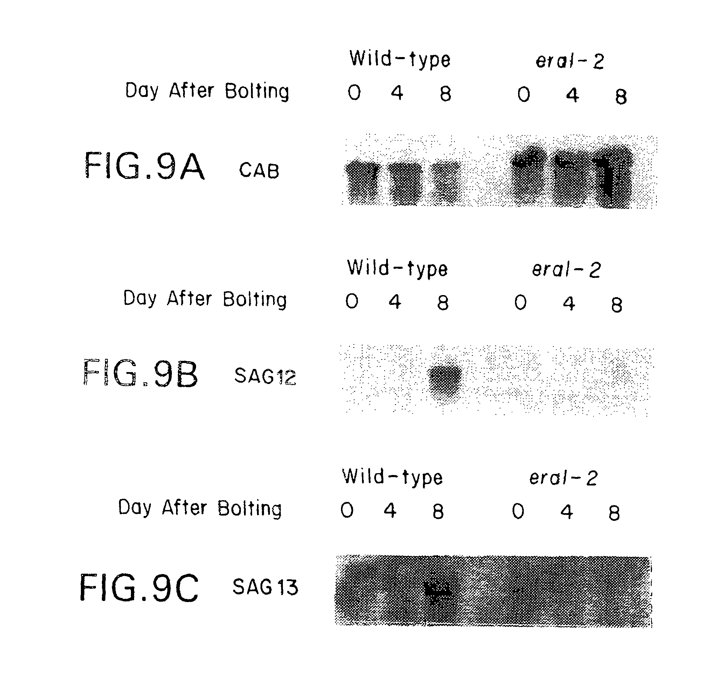

Moreover, under a 10 hr day/16 hr night cycle, the plant life cycle can be doubled versus the wild-type plants (3 months). It appears therefore, that chlorophyll turnover and senescence signals are altered in the eral mutant. For example, wild-type and mutant plants were grown in pots under well-watered conditions to stages of development where the wild-type plant leaves would begin to senesce (about the time of flower development). At this time, developmentally-similar leaves were assayed for senescence-induced marker genes by northern blot analysis (Example 8). Two genes, SAG12 and SAG13, in which transcription is normally induced during senescence in wild-type plants, were not induced in the *era*l mutant (Figure 9). Further, CAB transcription is maintained (Figure 9). Taken together, these results indicate the senescence induction program in *era1* mutants is delayed compared to wild-type plants, showing that loss of farnesylation activity causes a retardation of the induction of senescence in the plant even under conditions wherein water stress is not an environmental factor.

In addition to effects on senescence and water loss, the eral mutants show a difference in branching and flowering habit when grown under diurnal light cycles. Under continuous (24 hours light/day) light, the branching pattern of mutancs does not differ from that of wild-type plants. However, when given a dark period, the mutants do not produce as many lateral branches as wild-type plants. When measured, plants with loss of farnesylation activity produced only 2.4 branches per plant compared to 3.6 lateral branches per wild-type plant. This represents a 30% decrease in lateral branches per plant.

Flowering is affected by loss of Ftase activity as well. Plants lacking Ftase activity produce more flowers per plant (25-30 buds/inflorescence) than wild-type plants (10-15 buds/inflorescence). Thus, on average there are twice as many flower buds are present on the mutants than on the wild-type plants.

These pleiotrophic effects of the eral loss of function mutants on whole plant development indicate that the ERA1 gene can be a coordinate regulator of a collection of plant developmental functions.

Until now, there was no known function for farnesylation in higher plants, including a role in ABA signal transduction. Ftases have been found in a number of higher plants such as tomato and pea, so it is clear that this enzyme has functions across species boundaries. Furthermore, overproduction of farnesyl transferase target peptides or the use of farnesylation inhibitors completely inactivates Ftase in mammalian and yeast systems. Thus, similar inhibitors can be applied to higher plants to inactivate Ftase *in vivo*. In both cases with the appropriate promoters, antisense Ftase or peptide inhibitors can be specifically targeted and controlled.

The farnesylation deficient mutants are also supersensitive to exogenous auxin. That these mutants show reduced branching and minor alterations in meriscem organization, can be explained by altered auxin regulation. Thus other hormone functions are affected in this mutant, which indicates that, in addition to ABA pathways, other hormone regulated pathways are controlled by Ftase activity. These results demonstrate that the ERA1 gene provides a molecular mechanism to coordinate regulation of different hormone signaling molecules.

In accordance with the present invention, the plants included within the scope of this invention are higher and lower plants of the plant kingdom. Mature plants, seedlings and seeds are included in the scope of the invention. A mature plant includes a plant at any stage in development beyond the seedling. A seedling is a very young, immature plant in the early stages of development. Plant parts, protoplasts and tissue culture are also provided by this invention.

Transgenic plants are included within the scope of the present invention which have the phenotype characterized by the eral mutation. Seed of transgenic plants are provided by this invention and can be used to propagate more plants containing the constructs of this invention.

ERA1 function in a number of crop plants can be inhibited to enhance the plant's response to adverse environmental conditions that require ABA-mediated signaling. Control of farnesylation in higher plants regulates both embryonic and vegetative tissue response to this hormone (Cutler, *et al*., 1996). The increased sensitivity translates into a faster response of the tissue to stress conditions which in turn confers increased protection of the plant to the environmental stress. Because this only requires the control of a single gene, ERA1, it should be possible to control farnesylation in a variety of plants by controlling the synthesis or activity of this enzyme. Furthermore, the work described herein clearly indicates that altering the ABA signal transduction pathway by manipulating the genes that control the ABA response makes it possible to improve the plant's response to adverse water stress conditions.

To produce transgenic plants of this invention, a construct comprising the gene encoding Ftase, or nucleic acid encoding its functional equivalent, and a promoter are incorporated into a vector through methods known and used by those of skill in the art. The promoter can comprise all or part of SEQ ID NO:3. The construct can also include any other necessary regulators such as terminators or the like, operably linked to the coding sequence. It can also be beneficial to include a 5' leader sequence, such as the untranslated leader from the coat protein mRNA of alfalfa mosaic virus (Jobling, S.A. and Gehrke, L. (1987) *Natur*e *325*:622-625) or the maize chlorotic mottle virus (MCMV) leader (Lommel, S.A., *et al*. (1991) *Virology 81*:382-385). Those of skill in the art will recognize the applicability of other leader sequences for various purposes.

Targeting sequences are also useful and can be incorporated into constructs. A targeting sequence is usually translated into a peptide which directs the polypeptide product of the coding nucleic acid sequence to a desired location within the cell, such as to the plastid, and becomes separated from the peptide after transit of the peptide is complete or concurrently with transit. Examples of targeting sequences useful in this invention include, but are not limited to, the yeast mitochondrial presequence (Schmitz, *et al*. (1989) *Plant Cell 1*:783-791), the targeting sequence from the pathogenesis-related gene (PR-1) of tobacco (Cornellisen, *et al*. (1986) *EMBO J. 5*:37-40), vacuole targeting signals (Chrispeels, M.J. and Raikhel, N.V. (1992) *Cell 68*:613-616), secretory pathway sequences such as those of the ER or Golgi (Chrispeels, M.J. (1991) *Ann*. *Rev*. *Plant Physiol*. *Plant Mol*. *Biol*. *42*:21-53). Intraorganellar sequences may also be useful for internal sites, *e*.*g*., thylakoids in chloroplasts. Theg, S.M. and Scott, S.V. (1993) *Trends in Cell Biol*. *3*:186-190.

In addition to 5' leader sequences, terminator sequences are usually incorporated into the construct. In plant constructs, a 3' untranslated region (3' UTR) is generally part of the expression plasmid and contains a polyA termination sequence. The termination region which is employed will generally be one of convenience, since termination regions appear to be relatively interchangeable. The octopine synthase and nopaline synthase termination regions, derived from the Ti-plasmid of *A. tumefaciens*, are suitable for such use in the invention.

Any suitable technique can be used to introduce the nucleic acids and constructs of this invention to produce transgenic plants with an altered genome. For grasses such as maize, microprojectile bombardment (see for example, Sanford, J.C., *et al*., U.S. Patent No. 5,100,792 (1992) can be used. In this embodiment, a nucleotide construct or a vector containing the construct is coated onto small particles which are then introduced into the targeted tissue (cells) via high velocity ballistic penetration. The vector can be any vector which permits the expression of the exogenous DNA in plant cells into which the vector is introduced. The transformed cells are then cultivated under conditions appropriate for the regeneration of plants, resulting in production of transgenic plants.

Transgenic plants carrying the construct are examined for the desired phenotype using a variety of methods including but not limited to an appropriate phenotypic marker, such as antibiotic resistance or herbicide resistance, or visual observation of the time of floral induction compared to naturally-occurring plants.

Other known methods of inserting nucleic acid constructs into plants include Agrobacterium-mediated transformation (see for example Smith, R.H., *et al*., U.S. Patent No. 5,164,310 (1992)), electroporation (see for example, Calvin, N., U.S. Patent No. 5,098,843 (1992)), introduction using laser beams (see for example, Kasuya, T., *et al*., U.S. Patent No. 5,013,660 (1991)) or introduction using agents such as polyethylene glycol (see for example Golds, T. *et al*. (1993) *Biotechnology*, 11:95-97), and the like. In general, plant cells may be transformed with a variety of vectors, such as viral, episomal vectors, Ti plasmid vectors and the like, in accordance with well known procedures. The method of introduction of the nucleic acid into the plant cell is not critical to this invention.

The methods of this invention can be used with in planta or seed transformation techniques which do not require culture or regeneration. Examples of these techniques are described in Bechtold, N., *et al*. (1993) CR *Acad*. *Sci. Paris*/*Life Sciences 316:*116-93; Chang, S.S., *et al*. (1990) *Abstracts of the Fourth International Conference on Arabidopsis Research,* Vienna, p. 28; Feldmann, K.A. and Marks, D.M (1987) *Mol. Gen*. *Genet*. *208*:1-9; Ledoux, L., *et al*. (1985) *Arabidopsis Inf*. *Serv*. *22*:1-11; Feldmann, K.A. (1992) *In:* Methods in Arabidopsis Research (Eds. Koncz, C., Chua, N-H, Schell, J.) pp. 274-289; Chee, *et al*., U.S. patent, Serial No. 5,376,543.

The transcriptional initiation region may provide for constitutive expression or regulated expression. In addition to the ERA1 promoter, many promoters are available which are functional in plants.

Constitutive promoters for plant gene expression include, but are not limited to, the octopine synthase, nopaline synthase, or mannopine synthase promoters from *Agrobacterium*, the cauliflower mosaic virus (35S) promoter, the figwort mosaic virus (FMV) promoter, and the tobacco mosaic virus (TMV) promoter. Constitutive gene expression in plants can also be provided by the glutamine synthase promoter (Edwards, *et al*. (1990) *PNAS 87*:3459-3463), the maize sucrose synthetase 1 promoter (Yang, *et al*. (1990) *PNAS 87*:4144-4148), the promoter from the Rol-C gene of the TLDNA of Ri plasmid (Sagaya, *et al*. (1989) *Plant Cell* *Physiol*. *30*:649-654), and the phloem-specific region of the pRVC-S-3A promoter (Aoyagi, *et al*. (1988) *Mol*. *Gen. Genet. 213*:179-185).

Heat-shock promoters, the ribulose-1,6-bisphosphate (RUBP) carboxylase small subunit (ssu) promoter, tissue specific promoters, and the like can be used for regulated expression of plant genes. Developmentally-regulated, stress-induced, wound-induced or pathogen-induced promoters are also useful.

The regulatory region may be responsive to a physical stimulus, such as light, as with the RUBP carboxylase ssu promoter, differentiation signals, or metabolites. The time and level of expression of the sense or antisense orientation can have a definite effect on the phenotype produced. Therefore, the promoters chosen, coupled with the orientation of the exogenous DNA, and site of integration of a vector in the genome, will determine the effect of the introduced gene.

Specific examples of regulated promoters also include, but are not limited to, the low temperature Kinl and cor6.6 promoters (Wang, *et al*. (1995) *Plant Mol*. *Biol. 28*:605; Wang, *et al*. (1995) *Plant Mol*. *Biol*. *28*:619-634), the ABA inducible promoter (Marcotte Jr., *et al*. (1989) *Plant Cell 1*:969-976), heat shock promoters, such as the inducible hsp70 heat shock promoter of *Drosphilia melanogaster* (Freeling, M., *et al*. (1985) *Ann*. *Rev*. *of Genetics 19*: 297-323), the cold inducible promoter from *B*. *napus* (White, T.C., *et al*. (1994) *Plant Physiol*. *106*:917), the alcohol dehydrogenase promoter which is induced by ethanol (Nagao, R.T., *et al*., Miflin, B.J., Ed. *Oxford Surveys of Plant* *Molecular and Cell Biology*, Vol. 3, p 384-438, Oxford University Press, Oxford 1986), the phloem-specific sucrose synthase ASUS1 promoter from *Arabidopsis* (Martin, *et al*. (1993) *Plant J*. *4*:367-377), the ACS1 promoter (Rodrigues-Pousada, *et al*. (1993) *Plant Cell 5*:897-911), the 22 kDa zein protein promoter from maize (Unger, *et al*. (1993) *Plant Cell 5*:831-841), the ps1 lectin promoter of pea (de Pater, *et al*. (1993) *Plant Cell 5*:877-886), the phas promoter from *Phaseolus vulgaris* (Frisch, *et al*. (1995) *Plant J*. 7:503-512), the lea promoter (Thomas, T.L. (1993) *Plant Cell 5*:1401-1410), the E8 gene promoter from tomato (Cordes, *et al*. (1989) *Plant Cell 1*:1025-1034), the PCNA promoter (Kosugi, *et al.* (1995) *Plant J*. 7:877-886), the NTP303 promoter (Weterings, *et al*. (1995) *Plant J. 8*:55-63) , the OSEM promoter (Hattori, *et al*. (1995) *Plant J*. 7:913-925), the ADP GP promoter from potato (Muller-Rober, *et al*. (1994) *Plant Cell 6*:601-604), the Myb promoter from barley (Wissenbach, *et al*. (1993) *Plant J*. *4*:411-422), and the plastocyanin promoter from *Arabidopsis* (Vorst, *et al*. (1993) *Plant J*. *4*:933-945).

The vector can be introduced into cells by a method appropriate to the type of host cells (*e.g.*, transformation, electroporation, transfection). For the purposes of this disclosure, the terms "transformed with", "transformant", "transformation", "transfect with", and "transfection" all refer to the introduction of a nucleic acid into a cell by one of the numerous methods known to persons skilled in the art. Transformation of prokaryotic cells, for example, is commonly achieved by treating the cells with calcium chloride so as to render them "competent" to take up exogenous DNA, and then mixing such DNA with the competent cells. Prokaryotic cells can also be infected with a recombinant bacteriophage vector.

Nucleic acids can be introduced into cells of higher organisms by viral infection, bacteria-mediated transfer (*e*.*g*., *Agrobacterium* T-DNA delivery system), electroporation, calcium phosphate co-precipitation, microinjection, lipofection, bombardment with nucleic-acid coated particles or other techniques, depending on the particular cell type. For grasses such as corn and sorghum, microprojectile bombardment as described, for example, in Sanford, J.C., *et al*., U.S. Patent No. 5,100,792 (1992) can be used. Other useful protocols for the transformation of plant cells are provided in Gelvin *et al*., 1992. Suitable protocols for transforming and transfecting cells are also found in Sambrook *et al*., 1989. The nucleic acid constructs of this invention can also be incorporated into specific plant parts such as those described supra through the transformation and transfection techniques described herein.

To aid in identification of transformed plant cells, the constructs can be further manipulated to include genes coding for plant selectable markers. Useful selectable markers include enzymes which provide for resistance to an antibiotic such as gentamycin, hygromycin, kanamycin, or the like. Similarly, enzymes providing for production of a compound identifiable by color change such as GUS (β-glucuronidase), or by luminescence, such as luciferase, are useful.

For example, antisense Ftase can be produced by integrating a complement of the ERA1 gene linked to DNA comprising SEQ ID NO:3 into the genome of a virus that enters the host cells. By infection of the host cells, the components of a system which permits the transcription of the antisense present in the host cells.

When cells or protoplasts containing the antisense gene driven by a promoter of the present invention are obtained, the cells or protoplasts are regenerated into whole plants. The transformed cells are then cultivated under conditions appropriate for the regeneration of plants, resulting in production of transgenic plants. Choice of methodology for the regeneration step is not critical, with suitable protocols being available for many varieties of plants, tissues and other photosynthetic organisms. See, *e*.*g*., Gelvin S.B. and Schilperoort R.A., eds. *Plant Molecular Biology Manual*, *Second Edition*, Suppl. 1 (1995) Kluwer Academic Publishers, Boston MA, U.S.A.

Transgenic plants carrying the construct are examined for the desired phenotype using a variety of methods including but not limited to an appropriate phenotypic marker, such as antibiotic resistance or herbicide resistance as described *supra*, or visual observation of their growth compared to the growth of the naturally-occurring plants under the same conditions.

As used herein, the term transgenic plants includes plants that contain either DNA or RNA which does not naturally occur in the wild type (native) plant or known variants, or additional or inverted copies of the naturally-occurring DNA and which is introduced as described herein. Transgenic plants include those into which isolated nucleic acids have been introduced and their descendants, produced from seed, vegetative propagation, cell, tissue or protoplast culture, or the like wherein such alteration is maintained.

Such transgenic plants include, in one embodiment, transgenic plants which are angiosperms, both monocotyledons and dicotyledons. Transgenic plants include those into which DNA has been introduced and their progeny, produced from seed, vegetative propagation, cell, tissue or protoplast culture, or the like.

Seed can be obtained from the regenerated plant or from a cross between the regenerated plant and a suitable plant of the same species. Alternatively, the plant can be vegetatively propagated by culturing plant parts under conditions suitable for the regeneration of such plant parts.

The invention can be used to provide plant tissue culture and protoplasts which contain DNA comprising antisense or an altered ERA1 nucleic acid operably linked to an ERA1 promoter, which alters the response of the tissue culture or protoplasts to varying environmental conditions.

The methods of this invention can also be used with *in planta* or seed transformation techniques which do not require culture or regeneration. Examples of these techniques are described in Bechtold, N., *et al*. (1993) *CR Acad*. *Sci. Paris*/*Life Sciences 316*:118-93; Chang, S.S., *et al*. (1990) *Abstracts of the Fourth International Conference on Arabidopsis Research*, Vienna, p. 28; Feldmann, K.A. and Marks, D.M (1987) *Mol*. *Gen. Genet. 208*:1-9; Ledoux, L., *et al.* (1985) *Arabidopsis Inf*. *Serv*. *22*:1-11; Feldmann, K.A. (1992) *In*: Methods in *Arabidopsis* Research (Eds. Koncz, C., Chua, N-H, Schell, J.) pp. 274-289; Chee, *et al*., U.S. patent, Serial No. 5,376,543.

### EMBODIMENTS

The constructs and methods of this invention have numerous applications of commercial value, especially in the prevention of desiccation of plant tissues under periods of water stress. Genetic manipulation of crop plants incorporating inhibitors of Ftase or inactivation of the gene encoding endogenous plant Ftase would allow such plants to withstand transitory environmental stress and can broaden the environments where these plants can be grown. Thus, improving tolerance of crop plants to cold, salt and drought stress, can improve the yield of the plants under such adverse conditions.

The technology described herein can also be used to alter harvesting time and harvest quality of plants. For example, overexpression of Ftase could lead to faster drying times of crops, such as corn and other grasses. Drying corn involves the use of large amounts of propane gas. Drying times of crops such as hay, which dry naturally in the fields, could be shortened, making it less likely that rain would deteriorate the crop.

In addition, inhibition of farnesylation in plants can also be used to control the senescence program of the plants so that leaves can be maintained in a green state longer and fruits can be kept immature. For example, if an antisense construct of ERA1 or CaaX box inhibitor protein construct was placed under the control of a senescence-induced promoter, the plant would induce an inhibitor of farnesylation as the senescence program was initiated, which would in turn inhibit senescence. The result would be a plant which remains green or fruits which remain immature. Thus, the plant could be kept producing a product, such as a vegetative part, flower or fruit much longer. Thus, horticulturalists could produce plants which stayed green and continued to grow even though a wild-type plant of the same variety would senesce under the same conditions. Cut flowers could be maintained longer. Or a fruit could be kept immature, an important product for the vegetable industry where produce lifetime to market is extremely important.

Further, the inhibition of Ftase in fruits and vegetables can reduce wilting. Thus, wilting of produce during transport and shipping could be reduced. Fruits and vegetables on the grocery shelf would also require less misting to keep them fresh and flavorful, and there would be less need to wax produce such as cucumber, apples and oranges to keep them from drying out.

Less watering would also mean that fungal and bacterial attacks on the crops, or fruits and vegetables would be reduced. For example, plant diseases in the field which result from splashing of plant pathogens from the soil to the plant leaves and fruits could be inhibited.

In the field of horticulture, many drought-resistant varieties could be produced for landscaping and for use as ornamental house plants. Especially valuable would be varieties of plants which are used for potting, as ornamentals inside or outside homes and offices, and which can survive infrequent water. This would be a considerable boon for gardeners, especially during the droughty summer months where forgotten plants dry out quickly in the sun. Further, plants grown under trees and in other shady areas often experience drought conditions and limited light. The technology provided herein can provide plant varieties which can better survive under these conditions.

Horitculturalists could find many uses for plants wherein lateral branching and/or flower numbers can be regulated with light/dark cycles. Examples of plants in which longer, unbranched stems would confer marketable advantage include roses, carnations, lilies, and the like. The ability to increase the number of flowers or florets on the plant is also a highly valuable asset. These traits could also be useful for many agricultural crops in that yields can be increased in a manner which also made harvesting of the crop easier.

Another benefit of the constructs and methods provided herein is that the ERA1 promoter is active in the guard cells of leaves. A portion of the ERA1 gene promoter can be fused to antisense nucleic acid to the ERA1 gene so Ftase activity is diminished only in the guard cells.

A further embodiment is the use of the drought-resistant trait as a selectable marker of transformation in plants, plant cells and plant tissues. One method of detecting transformation in plants consists of: (a) incorporating a nucleic acid construct comprising a promoter operably-linked to nucleic acid comprising antisense to SEQ ID NO:1 or nucleic acid comprising a functional equivalent of the antisense; (b) inserting the nucleic acid construct into a plant, plant cell or plant tissue; (c) growing the plant, or regenerating a plant from the plant cell or plant tissue until stomates are formed; and (d) placing the plant or regenerated plant under conditions wherein the plant is drought stressed, wherein survival of the plant under drought conditions compared to untransformed plants is indicative of transformation. Thus, this technology can be used as a selectable genetic marker, *i*.*e*., a visual marker especially when combined with plant selection and transformation schemes.

In addition, without resorting to stressing a transgenic plant, the branching and/or flowering habit of plants with loss of Ftase function differs substantially from that of wild-type plants and can be used as a marker for successful transformation. This method would be especially useful where *in planta* transformation techniques have been applied. Under diurnal light conditions, shoots of transgenic plants will demonstrate less lateral branching than that of untransformed shoots, thus indicating effective loss of Ftase activity without the use of selective antibiotic markers.

### EXEMPLIFICATION

### Example 1: Mutagenesis conditions

*Arabidopsis* plants used in this study were grown under continuous light in soil or agar containing petri plates, as described elsewhere (Haughn and Somerville 1986) Two distinct wild-types of *Arabidopsis* were used: Meyerowitz's Colombia (Col) (Lelhe Seeds, Dripping Springs, TX) and Wassilewskija (Ws) (ABRC, Ohio State University). Mutants isolated by screening T-DNA mutagenized seeds were in the Wassilewskija background. These were obtained from the Ohio State Arabidopsis Seed stock collection (ABRC stock numbers CS2606-2654). The T-DNA seed collection was comprised of 49 pools of 1200 fourth generation (T4) offspring derived from 100 mutagenized parents. A mutagenized parent was obtained by incubating overnight wild-type seeds (T1) in a saturating *Agrobacterium* culture containing a T-DNA plasmid. The seeds were washed in water and planted into pots. T2 generation seed were obtained from each plant and tested for kanamycin resistance (which is carried on the T-DNA). Kanamycin-resistant plants were advanced to the T3 generation. T4 generation plants were given to the stock center. Each pool was screened separately.

Mutants isolated by screening fast neutron irradiated seeds were in Meyerowitz's Columbia background. Mutagenized wild-type seeds (N1) were irradiated with 60 Gy of fast neutrons and grown to the next generation. The N2 seeds were obtained as pools of approximately 11,000 seed generated from 1387 N1 parents. Ten of these pools were screened separately for ABA supersensitive mutations. In the initial screen the seeds had been stored at 4°C and were plated without imbibing. For all subsequent rescreening seeds were imbibed at 4°C for one week on 0.3 µM ABA and scored for cotyledon emergence after 5-7 days at 22°C in the light.

### Example 2: Genetic Analysis

Mutant lines were backcrossed to wild type three times. T-DNA mutations were backcrossed to Ws and fast neutron mutants to MCol. Segregation of the *era* phenotype was followed by plating F2 seeds on both 0.3 µM ABA and imbibing four days at 4°C. Following imbibition, plates were transferred to room temperature in the light. Germination was measured as the presence or absence of expanded cotyledons in seedlings one week post-imbibition. Double mutants were constructed by crossing lines homozygous for each mutation following segregation and identifying lines that carried one of the mutant phenotypes. The *abi*3 allele used in this study is *abi*3-6 (Nambara *et al*., 1994) and *abi*1 is *abi*1-1 (Koornneef *et al*., 1982). The *era*1-2 allele was used as the *era* parent. Segregation analysis suggested *era*1 partially suppressed the insensitivity of *abi*1 so F2 plants were first screened for insensitivity to 3 mM ABA and F3 seed from these plants were scored for sensitivity to 0.3 µM ABA. Putative double mutants were progeny tested in the F4 generation and verified by DNA polymorphisms for both Abi1 and Era1. For *era1* abi3 double mutants, F2 seeds were screened for insensitivity (3 µM ABA) and mature plants were scored for protruding carpels and immature green seeds (Nambara *et al*., 1994). Putative double mutant lines were verified by DNA polymorphisms for both Abi3 and Era1.

### Example 3: DNA and RNA Analysis

The methods for DNA (Dellaporta *et al*., 1983) and RNA (Verwoerd *et al*., 1989) extractions were as described. High stringency Southerns were carried out at 65C as described elsewhere (Sambrook *et al*., 1989). All screening of genomic and cDNA libraries were done on Gelman BioTrace NT membranes according to manufacturers specifications (Gelman Sciences). To clone insertion junctions between T-DNA and genomic DNA in the T12W mutant (*era*1-1) a library of T12W DNA was made in γ-ZAPII (Stratagene). Genomic Southern blots of T12W DNA digested with *Eco*R1 and probed with right border (RB) T-DNA produced three bands (13 Kb, 7.0 Kb and 8.0 Kb). Subsequent analysis with other restriction enzymes verified the 7 and 8 Kb bands as containing the insertion points of T-DNA and flanking plant DNA. These fragments were cloned by digesting genomic DNA with *Eco*RI and the DNA was fractionated using a Prep Cell (Pharmacia). Fractions containing the 7 and 8 Kb fragments were identified by Southern analysis of pooled fractions using the RB as a probe. These pools were ligated to the γ-ZAPII arms according to the manufacturer's instructions (Stratagene). A library of 40,000 recombinants was screened. Five positive plaques were identified and excised plasmid forms of the cloned inserts were isolated according to the manufacturer (Stratagene). Two plasmids, designated pL4B and pL7, which hybridized with RB probe were characterized further. Using pBluescript, a 2.3 Kb *Eco*RI *Bam*HI fragment from pL4B was subcloned and designated pSC10 and 1.3 Kb *Hind*III *Bam*HI fragment from pL7 was subcloned and designated pSC11. These two plasmids contain approximately 1.2 Kb of T-DNA attached to the flanking plant genomic DNA. pSC10 was used as a probe to screen an Arabidopsis cDNA library, PRL2 1-ZipLox (ABRC, Stock CD4-7). Five positive cDNA's were identified and the longest cDNA insert, pZL23, was used to screen an additional 200,000 PRL2 phage. From this screening a longer cDNA insert, pZL51, (1.35 Kb) was isolated. These clones were sequenced and used to screen 30,000 γ-ZAPII plaques made from partially digested *Eco*R1 wild-type Columbia genomic DNA. Construction of this library was as described above except without fractionation of the digested DNA. Four positive clones were identified. The inserts were excised and a 6 Kb region encompassing the pZL51 clone was completely sequenced. This genomic insert and a14 Kb genomic insert isolated from a 1-FIX Lansberg erecta genomic library by similar methods (ABRC Stock CD4-8) were used as probes to size deletions in the fast neutron mutants (*era*1-2, *era*1-3).

### Example 4: Protein Farnesyl transferase Assay

Farnesyl transferase (Ftase) assays were carried out using cell free extracts from wild-type and mutant planes as the Ftase source and synthetic heptapeptides as substrate for the reaction. Peptides were purchased from Genemed Biotechnologies, Inc. The appropriate sequences for the peptides were based on the data of Randall *et al*. (1993); these were GGCCAIM (-CAIM) and GGCCAIL(-CAIL). Solutions of peptides were prepared in 100% dimethyl sulfoxide (DMSO) containing 10 mM dithiotreitol (DTT) and diluted in 10 mM DTT in the absence of DMSO. The source of Ftase for transferase assays were soluble protein extracts from the buds of three week old plants. For both wild-type and mutant plants 1 g (fresh weight) of buds were collected and homogenized in a buffer containing 50 mM Hepes (pH 7.5), 1 mM MgCl₂, 1 mM EGTA, 5 mM DTT, 2 µg/ml leupeptin, 2 µg/ml aprotinin, and 1mM PMSF. Cellular debris and membranes were removed by centrifugation at 4°C at 10 000 g for 10 min and 100 000 g for 30 min. The supernatant was saved and quantification of total soluble protein was assessed according to Bradford (1976). Soluble extracts were incubated at 30°C with a peptide substrate and ³H-farnesyl pyrophosphate (Amersham) for 40 min. Each reaction mixture contained the following components in a final volume of 25 µl: 50 mM Hepes (pH 7.5), 5 mM MgCl₂, 5 mM DTT, 50 µM peptide, 0.5 µM [³H]FPP, and 100 µg of soluble protein extract. As a control one reaction contained soluble extracts that had been boiled for 5 min. Reactions were terminated by adding EDTA to a final concentration of 50 mM and then spotted onto Silica Gel 60 thin-layer chromatography plates (Millipore). Plates were developed with n-propanol-water (7:3 v/v) for 4-5 h. The plates were dried, sprayed with En³Hance (New England Nuclear), and exposed to Kodak X-OMAT AR film at -70°C for 4 days.

### Example 5: ERA1-GUS gene constructs and transgenic plants

ERA1-GUS fusion constructs were made by inserting a 5 Kb EcoR1-HindIII genomic fragment of the ERA1 promoter in to promoterless GUS T-DNA plasmid (pBI121). This construct was transformed into Agrobacterium strain LB4404. The *Agrobacterium* was grown to (0.8 O.D. units, 595 nm) and then washed extensively in water, cells were resuspended in 10% glycerol and then pulsed in an electroporator at 200 Ohms 25 µF, 2.5 kvolts. Cells were then placed on L3 media plus Ampicillin (100 µg/ml) and grown for 2 days at 28°C. Ampicillin-resistant transformants were used in subsequent plant transformation experiments. Transgenic plants were made by vacuum infiltrating plants with a saturating *Agrobacterium* culture (0.8 O.D. units, 595 nm). Wild-type plants were grown under standard laboratory conditions (25°C, 150 µE m⁻² sec ⁻¹, humidity, constant light) until they produced their first bolts at approximately 5 weeks. Stems were removed and the plants were submerged in a solution of *Agrobacterium* and placed under vacuum (20 mBars) for 5 minutes. After the vacuum was broken plants were transferred to soil and allowed to recover under standard laboratory conditions. Plants produced new flowers and seed which was harvested after 2 months and allowed to dry for 2 weeks. Seed from individual plants were planted onto minimal media MS plates containing 50 µg/ml Kanamycin. Green kanamycin-resistant plantlets were identified and moved to soil after 2 weeks and allowed to grow for seed. These seeds were germinated and the seedlings tested for GUS activity using the fluorescent GUS substrate Imagene Green (Molecular Probes, Eugene, Oregon). The assay was done by suspending seedlings in GUS-buffer (50 mM Sodium phosphate pH 7.0, 10 mM EDTA, 0.1% Triton X-100, 0.1% Sodium sarcosyl, 4 mM Imagene Green) for 2-4 hours in the dark at room temperature. Seedlings were directly viewed under a microscope (25X) using blue excitation light for a positive fluorescent signal which is yellow on a red chlorophyll autofluorescent background.

### Example 6: Drought Experiments.

Six single wild-type and six *era*1-2 seedlings were grown for four weeks in constant light with constant watering (25°C, 150 µE m⁻² sec⁻¹ , 70% humidity, constant light). Pots were then covered in tinfoil to retard soil evaporation and the plant and pot was weighed. At this time, plants were no longer watered and every day each pot was weighed. At the end of the experiment plants were removed and the pots were allowed to dry another two weeks and then weighed to determine the weight of the dry soil and pot. This weight was subtracted from each sample.

### Example 7: Age-related changes in detached leaves.

The chlorophyll content in adult rosette leaves in wild-type Columbia and era1-2 mutants were compared after detachment from these *Arabidopsis* plants. The plants were grown under constant light (150 µEinsteins/m² sec) and temperature (22°C) to a similar developmental age (3 weeks following germination). At this time, the fifth leaves of several plants which had emerged after germination were removed and placed on 0.8% agar containing minimal salts in petri plates. The plates were sealed and placed at 22°C under constant light (50 µEinsteins/m²·sec) for 12 days. Photographs were taken and color comparisons made at 0, 3, 6, 9, and 12 days.

### Example 8: Determination of transcript levels for selected Genes in aging leaves.

Plants were grown under constant light (150 µEinsteins/m²·sec) and temperature (22°C) to a similar developmental age (4 weeks following germination), at which time the fifth rosette leaf which had emerged following germination was removed from mutant (era1-2) and wild-type plants. These leaves were assayed for the expression of three genes (CAB, SAG12, SAG13) by northern analysis (0, 4, 8 days after the plants bolted). The CAB gene encodes the Arabidopsis chlorophyll binding protein which is involved in capturing light for photosynthesis. CAB is required for the green color of the leaf and is a good marker of chlorophyll turnover in the plant. CAB in wild-type plants shows transcript level reduction once senescence is induced. No transcript level reduction was observed in aging leaves of *era*1-2 mutants. SAG12 and SAG13 are *Arabidopsis* genes cloned by differential expression during senescence (SAG stands for senescence Activated Gene) . Transcription of both genes is induced during the onset of senescence in wild-type *Arabidopsis* plants. It was determined that these genes were not induced under the same developmental conditions in the era1-2 mutants.

### References

Baskin, JM and Baskin, CC (1971) Can J Bot 50:277.

Chandler, PM and Robertson, M (1994) Gene expression regulated by abscisic acid and its relationship to stress tolerance. Ann Rev Plant Physiol and Plant Mol Biol 45:113-141.

Chen, W-J, Anders, DA, Goldstein, JL, Russell, DW, Brown, MS (1991) Cell 66:327

Cutler, S, Ghassemian, M, Bonetta, D, Cooney, S, McCourt, P (1996) A protein farnesyl transferase involved in abscisic acid signal transduction in Arabidopsis. Science 273:1239-1241.

Dellaporta, S. L., Wood, J. and Hicks, J. B. (1983). A plant DNA minipreparation: version II. Plant Mol. Biol. Rep. 1:19-21.

Eisenmann, D. M. and Kim, S. K. (1994). Signal transduction and cell fate specification during *Caenorhabditis elegans* vulval development. Curr. Opin. Genet. Dev. 4:508-516.

Ellington, A. (1987). Preparation and Analysis of DNA. In Current Protocols in Molecular Biology F. Ausubel *et al*. eds. (Boston, Greene). pp 2.0.1-2.12.5.

Goodman, LE, Perou, CM, Fujiyama, A, Tamanoi, F (1988) Yeast 4:271

Haughn, G. and Somerville C. R. (1986). Sulfonylurea-resistant mutants of Arabidopsis thaliana. Mol. Gen. Genet. 204:430-434.

Koornneef, M, Reuling, G and Karssen, CM (1984) The isolation and characterization of abscisic acid-insensitive mutants of *Arabidopsis thaliana*. Physiol. Plant. 61:377-383.

Leung, J, Bouvier-Durand, M, Morris, P-C, Guerrier, D, Chefdor, F, and Giraudat, J (1994) *Arabidopsis* ABA-response gene ABI1: features of a calcium-modulated protein phosphatase. Science 264:1448-1452.

Meyer, K, Leube, MP, and Grill, E (1994) A protein phosphatase 2C involved in ABA signal transduction in *Arabidopsis thaliana*. Science 264:1452-1455.

Randall, SK, Marshall, MS, Crowell, DN (1993) Protein isoprenylation in suspension-cultured tobacco cells. Plant Cell 5:433-442.

Reid, JB, and Howell, SH (1995) The function of hormones in plant growth and development. In Plant Hormones Physiology, Biochemistry and Molecular Biology. 2^{nd} ed. P. Davies ed. (Dortrecht Kluwer) pp. 448-485.

Sambrook, J., E. F. Fritsch and Maniatis, T. (1989). Molecular Cloning: A Laboratory Manual, Second edition (Cold Spring Harbor, New York: Cold Spring Harbor Laboratory Press)

Schafer, WR, and Rine, J (1992) Protein Prenylation: Genes, Enzymes, Targets and Functions. Ann Rev Genet 30:209-237.

Shirley, BW, Hanley, S, Goodman, HM (1992) Plant Cell 4: 333

Verwoerd, T. C., Dekker, B. M. M. and Hoekema, A. (1989). A small-scale procedure for the rapid isolation of plant RNA's. Nucleic Acids Research 17:2362.

Yang, Z, Cramer, CL, and Watson, JC (1993) Protein farnesyl transferase in plants. Plant Physiology 101:667-674.

## Claims

1. A method of producing a drought-tolerant plant comprising:
a) providing a nucleic acid construct comprising a promoter operably-linked to an antisense nucleic acid of a nucleic acid sequence encoding a beta subunit of a famesyl transferase polypeptide;
b) inserting said nucleic acid construct into a vector;
c) transforming a plant, tissue culture, or a plant cell with the vector and
d) growing the plant or regenerating a plant from the tissue culture or plant cell;
wherein a drought-tolerant plant is produced.

2. The method of claim 1, wherein said antisense nucleic acid comprises 25 or more consecutive nucleic acids complementary to SEQ ID NO:1 shown in Figure 1.

3. The method of claim 1, wherein said promoter is a guard cell specific promoter.

4. The method of claim 1, wherein said promoter is the era-1 promoter having SEQ ID NO:3 shown in Figure 3.

5. A drought tolerant transgenic plant obtainable by the method of any one of claims 1 to 4.

6. A transgenic seed obtainable by the transgenic plant of claim 5, wherein said seed produces a drought tolerant plant.

## Patentansprüche

1. Verfahren zur Herstellung einer gegenüber Trockenheit toleranten Pflanze, umfassend:
a) Bereitstellen eines Nukleinsäurekonstrukts, das einen Promotor umfasst, der mit einer Antisense-Nukleinsäure einer Nukleinsäuresequenz wirksam verbunden ist, die eine Beta-Untereinheit eines Farnesyltransferasepolypeptids kodiert;
b) Einbauen des Nukleinsäurekonstrukts in einen Vektor;
c) Transformieren einer Pflanze, einer Gewebekultur oder einer Pflanzenzelle mit dem Vektor; und
d) Züchten der Pflanze oder Regenerieren einer Pflanze aus der Gewebekultur oder der Pflanzenzelle, wobei eine gegenüber Trockenheit tolerante Pflanze hergestellt wird.

2. Verfahren nach Anspruch 1, wobei die Antisense-Nukleinsäure 25 oder mehr aufeinanderfolgende Nukleinsäuren umfasst, die zu der in Figur 1 gezeigten SEQ ID Nr. 1 identisch sind.

3. Verfahren nach Anspruch 1, wobei der Promotor ein Schließzellen spezifischer Promotor ist.

4. Verfahren nach Anspruch 1, wobei der Promotor der era-1 Promotor ist, der die in Figur 3 gezeigte SEQ ID Nr. 3 aufweist.

5. Transgene, gegenüber Trockenheit tolerante Pflanze, die durch das Verfahren nach einem der Ansprüche 1 bis 4 erhältlich ist.

6. Transgener Samen, der durch die transgene Pflanze von Anspruch 5 erhältlich ist, wobei der Samen eine gegenüber Trockenheit tolerante Pflanze erzeugt.

## Revendications

1. Procédé pour l'obtention d'une plante tolérant la sécheresse, comprenant les étapes consistant à :
a) procurer une construction d'acides nucléiques comprenant un promoteur lié de manière opérante à un acide nucléique antisens d'une séquence d'acides nucléiques encodant une sous-unité bêta d'un polypeptide de farnésyl transférase ;
b) insérer ladite construction d'acides nucléiques dans un vecteur ;
c) transformer une plante, une culture tissulaire ou une cellule végétale avec le vecteur ; et
d) faire croître la plante ou régénérer une plante à partir de la culture tissulaire ou de la cellule végétale ;
dans lequel on obtient une plante tolérant la sécheresse.

2. Procédé selon la revendication 1, dans lequel ledit acide nucléique antisens comprend 25 acides aminés successifs ou plus complémentaires à la séquence SEQ ID NO : 1 représentée en figure 1.

3. Procédé selon la revendication 1, dans lequel ledit promoteur est un promoteur spécifique du stomate.

4. Procédé selon la revendication 1, dans lequel ledit promoteur est le promoteur era-1 possédant la séquence SEQ ID NO : 3 représentée en figure 3.

5. Plante transgénique tolérant la sécheresse que l'on obtient via le procédé selon l'une quelconque des revendications 1 à 4.

6. Semence transgénique que l'on obtient via la plante transgénique selon la revendication 5, ladite semence produisant une plante tolérant la sécheresse.
